(19) Europäisches Patentamt
European Patent Office
Office européen des brevets

(11) Veröffentlichungsnummer: **0 023 257**
**B1**

(12) **EUROPÄISCHE PATENTSCHRIFT**

(45) Veröffentlichungstag der Patentschrift: **22.09.82**

(51) Int. Cl.$^3$: **C 07 C 91/44**, A 61 K 7/13

(21) Anmeldenummer: **80103229.3**

(22) Anmeldetag: **11.06.80**

# (54) Dichlor-Hydroxyethylaminophenole, Verfahren zu deren Herstellung, deren Verwendung und sie enthaltende Haarfärbemittel.

(30) Priorität: **15.06.79 DE 2924089**

(43) Veröffentlichungstag der Anmeldung: **04.02.81 (Patentblatt 81/05)**

(45) Bekanntmachung des Hinweises auf die Patenterteilung: **22.09.82 Patentblatt 82/38**

(84) Benannte Vertragsstaaten: **AT BE CH DE FR GB IT LI NL SE**

(56) Entgegenhaltungen:
**DE A 2 509 096**
**DE C 744 757**
**FR A 2 315 255**
**FR A 2 348 911**
**US A 4 031 160**
**US A 4 035 422**

(73) Patentinhaber: **Henkel Kommanditgesellschaft auf Aktien**
**Postfach 1100 Henkelstrasse 67**
**D-4000 Düsseldorf 1 (DE)**

(72) Erfinder: **Rose, David, Dr.**
**Holbeinweg 7**
**D-4010 Hilden (DE)**
Erfinder: **Lieske, Edgar**
**Hunsrückenstrasse 40**
**D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 257 B1

Imprimerie Jouve, 18, rue St-Denis, 75001 Paris, France

# Dichlor-Hydroxyethylaminophenole, Verfahren zu deren Herstellung, deren Verwendung und sie enthaltende Haarfärbemittel

Gegenstand der Erfindung sind neue Dichlor-hydroxyethylaminophenole der allgemeinen Formel

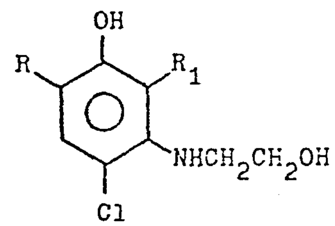

in der jeweils R oder $R_1$ ein Chloratom und der andere Rest Wasserstoff darstellt.

Die Herstellung der neuen Dichlor-hydroxyethylaminophenole erfolgt in 3 Stufen. Zunächst wird in erster Stufe ein entsprechend substituiertes Dichloraminophenol mit β-Chlorethyl-chlorformiat zu dem entsprechenden β-Chlorethyl-N-(dichlorhydroxyphenyl)-carbamat umgesetzt. Hieraus wird in zweiter Stufe durch Behandlung mit Lauge unter Ringschluß das entsprechende N-(Dichlorhydroxyphenyl)-oxazolidin-2-on hergestellt. In dritter Stufe wird aus dem Oxazolidin-2-on unter Kohlendioxidabspaltung das entsprechende Dichlorhydroxyethylaminophenol erhalten. Die Reaktion verläuft dabei nach folgendem Schema :

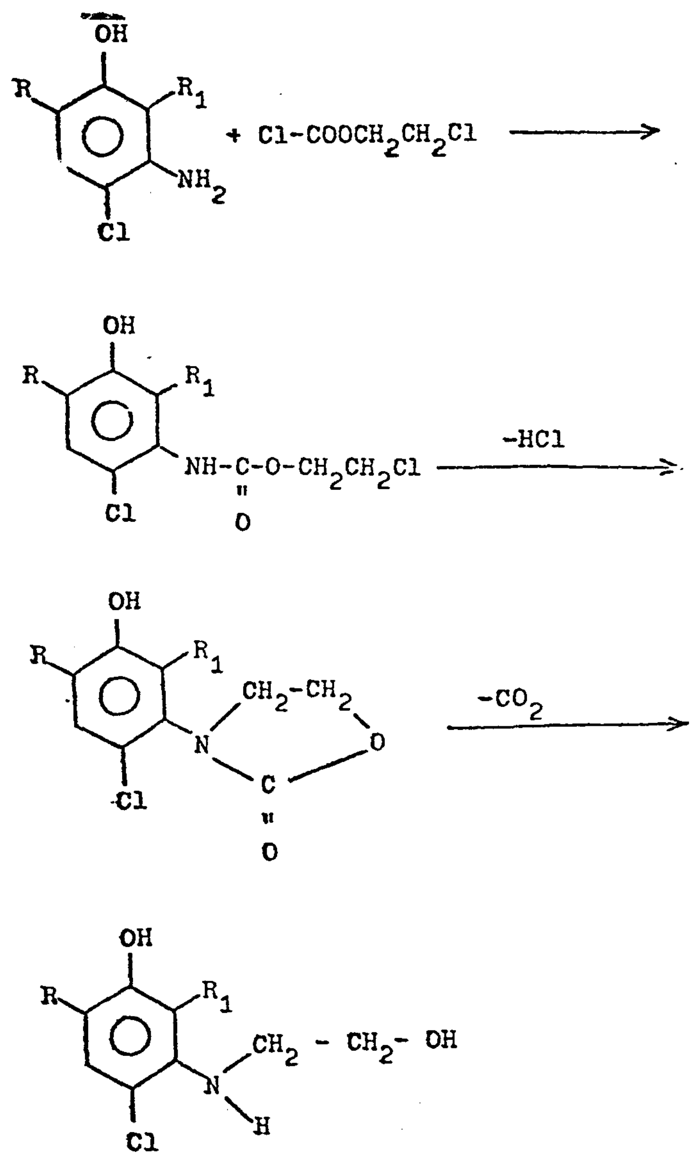


R beziehungsweise $R_1$ = Cl

Weitere Gegenstände der Erfindung sind die Verwendung der neuen Dichlor-hydroxyethylaminophenole als solcher oder in Gestalt ihrer Salze mit anorganischen oder organischen Säuren als Kupplertkomponenten in Oxidationshaarfarben sowie Haarfärbemittel, die die neuen Dichlorhydroxyethylaminophenole bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Kupplerkomponenten Dichlorhydroxyethylaminophenole der allgemeinen Formel

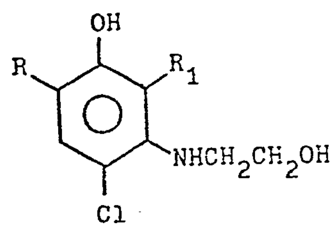

in der jeweils R oder $R_1$ ein Chloratom und der andere Rest Wasserstoff darstellt, sowie deren Salze mit anorganischen oder organischen Säuren in Kombination mit üblichen Entwicklersubstanzen verrwendet.

Haarfärbemittel auf Basis von Oxidationsfarbstoffen mit einem Gehalt an Dichlor-hydroxyethylaminophenolen der allgemeinen Formel

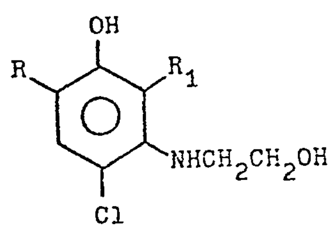

in der jeweils R oder $R_1$ ein Chloratom und der andere Rest Wasserstoff darstellt, sowie deren Salzen mit anorganischen oder organischen Säuren als Kupplerkomponenten und den in Oxidationshaarfarben üblichen Entwicklersubstanzen stellen demnach besonders wertvolle Kompositionen auf dem Gebiet der Oxidationshaarfarben dar.

Bei ihrem Einsatz als Kupplerkomponenten liefern die erfindungsgemäßen Verbindungen mit den allgemeinen für die Oxidationshaarfärbung verwendeten Entwicklersubstanzen sehr intensive, von türkis bis blauschwarz reichende Farbtöne und stellen somit eine wesentliche Bereicherung der oxidativen Haarfärbemöglichkeiten dar. Darüber hinaus zeichnen sich die erfindungsgemäßen Dichlorhydroxyethylaminophenole durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwendenden Dichlor-hydroxyethylaminophenole können entweder als solche oder in Form ihrer Salze mit anorganischen oder organischen Säuren, wie zum Beispiel als Chloride, Sulfate, Phosphate, Acetate, Propionate, Lactate, Citrate eingesetzt werden.

Als erfindungsgemäß einzusetzende Kupplerkomponenten sind 4,6-Dichlor-3-(β-hydroxyethylamino)-phenol und 2,4-Dichlor-3-(β-hydroxyethylamino)-phenol, sowie deren Salze mit anorganischen

oder organischen Säuren, zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino-6-piperidinopyrimidin, 2,4,5-Triamino-6-anilino-pyrimidin, 2,4,5-Triamino-6-morpholino-pyrimidin, 2,4,5-Triamino-6-β-hydroxy-ethylamino-pyrimidin anzuführen.

Zur Erzielung möglichst kräftiger und den natürlichen Haarfarbennuancen weitgehend entsprechender Farbtöne ist ein vollwertiger Blaufarbstoff als Nuancierkomponente von besonderer Wichtigkeit. Bei der Erstellung natürlicher Farbnuancen unter Zuhilfenahme von Blaukupplerkomponenten ergeben sich mit den üblichen Blaukupplern häufig Schwierigkeiten. Diesem Mangel kann durch den Einsatz der erfindungsgemäß zu verwendenden Dichlor-hydroxyethylaminophenole weitgehend abgeholfen werden. Diese Kupplerkomponenten liefern mit entsprechenden Entwicklersubstanzen neben anderen Farbnuancen dunkelblaue bis schwarzblaue, besonders intensive Haarfärbungen, die sich durch außerordentliche Lichtechtheiten auszeichnen.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden Dichlor-hydroxyethylaminophenole darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Perfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von circa 30 Minuten wird das Haarfärbemittel vom zu Färbenden Haar durch Spülen entfernt. Hernach wird das Haar mit einem milden Shampoo nachgewaschen und getrocknet.

Die mit den erfindungsgemäßen Haarfärbemitteln erzielbaren Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird nachstehend die Herstellung der erfindungsgemäßen Dichlor-hydroxyethylaminophenole beschrieben.

A) 4,6-Dichlor-3-(β-hydroxyethylamino)-phenol

1. Stufe : Herstellung von β-Chlorethyl-N-(2,4-dichlor-5-hydroxyphenyl)-carbamat.

58,6 g (0,33 Mol) 4,6-Dichlor-3-aminophenol wurden in 160 ml Dioxan gelöst und mit 18 g (0,18 Mol) Calciumcarbonat versetzt. Die erhaltene Suspension wurde auf 90 °C erwärmt und anschließend wurden unter Rühren 52 g (0,36 Mol) β-Chlorethyl-chlorformiat zugegeben. Nach beendeter Zugabe wurde noch 1 Stunde bei 90 °C weitergerührt. Nach dem Abkühlen wurde abfiltriert und die Mutterlauge mit 1 Liter Eiswasser versetzt. Das erhaltene β-Chlorethyl-N-(2,4-dichlor-5-hydroxyphenyl)-carbamat wurde abgesaugt, mit Wasser nachgewaschen und getrocknet. Der Schmelzpunkt des Produktes lag bei 130-131 °C.

2. Stufe : Herstellung von H-(2,4-Dichlor-5-hydroxyphenyl)-oxazolidin-2-on.

87 g β-Chlorethyl-N-(2,4-dichlor-5-hydroxyphenyl)-carbamat wurden in 233 ml einer 4,3 n Natronlauge, die auf 45 °C erwärmt war, eingerührt. Es wurde noch 20 Minuten bei gleicher Temperatur weitergerührt und danach die Reaktionslösung mit 200 ml Eiswasser versetzt. Nach Neutralisation mit verdünnter Salzsäure wurde das N-(2,4-Dichlor-5-hydroxyphenyl-oxazolidin-2-on abgesaugt und getrocknet. Das Produkt besitzt einen Schmelzpunkt von 234-236 °C.

3. Stufe : Herstellung von 4,6-Dichlor-3-(β-hydroxyethylamino)-phenol.

42,2 g (0,17 Mol) N-(2,4-Dichlor-5-hydroxyphenyl)-oxazolidin-2-on wurden bei 70 °C in 100 ml 5 n Natronlauge eingerührt. Nach 30 Minuten wurde die Reaktionsmischung auf 0 °C abgekühlt, durch Zugabe von Essigsäure neutralisiert und das Reaktionsprodukt abgesaugt und getrocknet. Das erhaltene 4,6-Dichlor-3-(β-hydroxyethylamino)-phenol besitzt einen Schmelzpunkt von 117-120 °C.

B) 2,4-Dichlor-3-(β-hydroxyethylamino)-phenol

1. Stufe : Herstellung von β-Chlorethyl-N-(2,6-dichlor-5-hydroxyphenyl)-carbamat.

Die Herstellung erfolgte völlig analog der 1. Stufe von Produkt A, wobei von 2,4-Dichlor-3-aminophenol ausgegangen wurde. Das erhaltene β-Chlorethyl-N-(2,6-dichlor-5-hydroxyphenyl)-carbamat stellt ein Öl dar.

2. Stufe : Herstellung von N-(2,6-Dichlor-5-hydroxyphenyl)-oxazolidin-2-on.

Das Produkt wurde völlig analog der 2. Stufe von Produkt A hergestellt, wobei als Ausgangsstoff β-Chlorethyl-N-(2,6-dichlor-5-hydroxyphenyl)-carbamat diente. Das erhaltene N-(2,6-Dichlor-5-hydroxyphenyl)-oxazolidin-2-on schmilzt bei 175-178 °C.

3. Stufe : Herstellung von 2,4-Dichlor-(β-hydroxyethylamino)-phenol.

Die Herstellung wurde völlig analog der 3. Stufe von Produkt A vorgenommen, wobei als Ausgangsstoff N-(2,6-Dichlor-5-hydroxyphenyl-oxazolidin-2-on diente. Das erhaltene 2,4-Dichlor-3-(β-hydroxyethylamino)-phenol besitzt einen Schmelzpunkt von 110-115 °C.

Die vorgenannten, in ihrer Herstellung beschriebenen Dichlorhydroxyethylaminophenole A) und B) wurden als Kupplerkomponenten in den folgenden Beispielen eingesetzt.

Als Entwicklerkomponenten dienten folgende Substanzen :

E1 : p-Phenylendiamin
E2 : 2,5-Diaminotoluol
E3 : N-Ethyl-N-(β-hydroxyethyl)-p-phenylendiamin
E4 : 1-Phenyl-3-carbamoyl-4-aminopyrazolon-5
E5 : N-Methyl-pyrrolidon-2-hydrazon
E6 : 2-Chlor-p-phenylendiamin
E7 : N,N-Dimethyl-p-phenylendiamin
E8 : $N^4$,$N^4$. Diethyl-2-methyl-p-phenylendiamin
E9 : 2,5-Diaminoanisol
E10 : N-Butyl-N-sulfobutyl-p-phenylendiamin
E11 : 2,4,5,6-Tetraaminopyrimidin.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}$-$C_{18}$,
10 Gew.-Teilen Fettalkoholsulfat (Natriumsalz) Kettenlänge $C_{12}$-$C_{18}$ und
75 Gew.-Teilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit Zusatz von Oxidationsmitteln wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Entwickler | Kuppler | Erhaltener Farbton mit 1 %iger $H_2O_2$-Lösung |
|---|---|---|---|
| 1 | E 1 | A | violettbraun |
| 2 | E 2 | A | schwarzviolett |
| 3 | E 3 | A | blauschwarz |
| 4 | E 4 | A | dunkelviolett |
| 5 | E 5 | A | goldgelb |
| 6 | E 6 | A | dunkelmagenta |
| 7 | E 7 | A | dunkelblau |
| 8 | E 8 | A | dunkelblau |
| 9 | E 9 | A | dunkelblau |
| 10 | E10 | A | dunkelblau |
| 11 | E11 | A | braunviolett |
| 12 | E11 | B | hellbraun |
| 13 | E 2 | B | braunviolett |

**Ansprüche**

1. Dichlor-hydroxyethylaminophenole der allgemeinen Formel

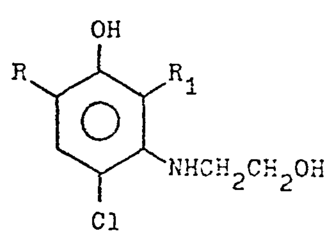

in der jeweils R oder $R_1$ ein Chloratom und der andere Rest Wasserstoff darstellt.

2. Verfahren zur Herstellung der Dichlor-hydroxyethyl-aminophenole gemäß Anspruch 1 dadurch gekennzeichnet, daß man in erster Stufe ein entsprechendes Dichloraminophenol mit β-Chlorethyl-chlorformiat zu dem entsprechenden β-Chlorethyl-N-(dichlorhydroxyphenyl)-carbamat umsetzt, hieraus in zweiter Stufe unter Ringschluß das entsprechende N-(Dichlorhydroxyphenyl)-oxazolidin-2-on bildet und aus diesem in dritter Stufe unter Kohlendioxidabspaltung das entsprechende Dichlorhydroxyethylaminophenol herstellt.

3. Verwendung von Dichlorhydroxyethylaminophenolen nach Anspruch 1 und 2, sowie deren Salzen mit anorganischen oder organischen Säuren als Kupplerkomponenten in Oxidationshaarfarbstoffen.

4. Haarfärbemittel auf Basis von Oxidationsfarbstoffen gekennzeichnet durch einen Gehalt an Dichlor-hydroxyethylaminophenolen gemäß Anspruch 1 und 3, sowie deren Salzen mit anorganischen oder organischen Säuren als Kupplerkomponenten und den in Oxidationshaarfarben üblichen Entwicklersubstanzen.

5. Haarfärbemittel nach Anspruch 4, gekennzeichnet durch einen Gehalt an Entwickler-Kuppler-Kombination in einer Menge von 0,2 bis 5 Gewichtsprozent, vorzugsweise 1 bis 3 Gewichtsprozent, bezogen auf das gesamte Haarfärbemittel.

**Claims**

1. Dichlorohydroxyethylaminophenols corresponding to the following general formula

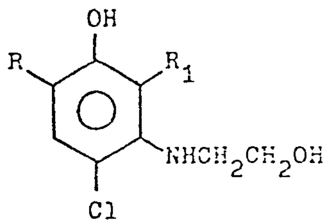

in which R or $R_1$ represents a chlorine atom and the other radical is hydrogen.

2. A process for producing the dichlorohydroxyethylaminophenols claimed in Claim 1, characterised in that, in a first step, a corresponding dichloroaminophenol is reacted with β-chloroethyl chloroformate to form the corresponding β-chloroethyl-N-(dichlorohydroxyphenyl)-carbamate from which the corresponding N-(dichlorohydroxyphenyl)-oxazolidin-2-one is formed by ring closure in a second step, the corresponding dichlorohydroxyethylaminophenol being produced therefrom in a third step in which carbon dioxide is eliminated.

3. The use of the dichlorohydroxyethylaminophenols claimed in Claims 1 and 2 and their salts with inorganic or organic acids as coupler components in oxidation hair dyes.

4. Hair dyes based on oxidation dyes, characterised by a content of dichlorohydroxyethylaminophenols of the type claimed in Claims 1 and 3 and their salts with inorganic or organic acids as coupler components and the developer substances normally used in oxidation dyes.

5. Hair dyes as claimed in Claim 4, characterised by a content of developer-coupler combination of from 0.2 to 5 % by weight and preferably from 1 to 3 % by weight, based on the hair dye as a whole.

**Revendications**

1. Dichloro-hydroxyéthylaminophénols de formule générale :

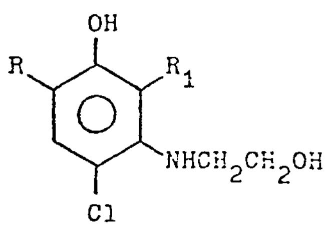

dans laquelle chaque fois R ou $R_1$ représente un atome de chlore et l'autre radical de l'hydrogène.

2. Procédé de préparation des dichlorohydroxyéthylaminophénols selon la revendication 1, caractérisé en ce que dans un premier stade on fait réagir un dichloraminophénol approprié avec du chloroformiate de β-chloréthyle pour obtenir le carbamate de β-chloréthyl-N-(dichlorohydroxyphényle) correspondant à partir duquel, dans un deuxième stade, on forme avec cyclisation la N-(dichlorohydroxyphényl)-oxazolidine-2-one correspondante et, à partir de celle-ci, dans un troisième stade, on prépare le dichloro-hydroxyéthylaminophénol correspondant par clivage de dioxyde de carbone.

3. Utilisation de dichloro-hydroxyéthylaminophénols selon les revendications 1 et 2, ainsi que leurs sels avec des acides minéraux ou organiques, comme composants de couplage dans des colorants d'oxydation pour cheveux.

4. Agents de teinture pour cheveux à base de colorants d'oxydation, caractérisés par une teneur en dichloro-hydroxyéthylaminophénols selon les revendications 1 et 3, ainsi que leurs sels avec des acides minéraux ou organiques comme composants de couplage et en les substances de développement en usage dans les teintures des cheveux par oxydation.

5. Agents de teinture pour cheveux selon la revendication 4, caractérisés par une teneur en la combinaison agent de développement-coupleur en une quantité de 0,2 à 5 % en poids, de préférence de 1 à 3 % en poids par rapport à l'agent de teinture pour cheveux total.